# EUROPEAN PATENT APPLICATION

(11) **EP 2 662 018 A1**
(43) Date of publication of application: **13.11.2013**
(21) Application number: 13167529.0
(22) Date of filing: 13.05.2013
(51) Int. Cl.: A61B 5/00

(54) **Optical Coherence Tomography Apparatus for Diagnosing Breast Cancer and Method of Controlling same**

(30) Priority: 11.05.2012 KR 20120050467
(71) Applicant: Samsung Electronics Co., Ltd, Gyeonggi-do 443-742 (KR)
(72) Inventor: Jang, Woo-young, Gyeonggi-do (KR); Choi, Hyun, Gyeonggi-do (KR)
(74) Representative: Grünecker, Kinkeldey, Stockmair & Schwanhäusser

(57) **Abstract**

An optical coherence tomography (OCT) apparatus and method thereof to diagnose breast cancer include an interference meter configured to split a light into a measurement light beam and a reference light beam to produce an interference between the reference light beam and a response light beam. A response light beam includes the measurement light beam reflected inside of a mammary duct. The optical probe is inserted into the mammary duct, irradiates the measurement light beam inside the mammary duct, and receives the response light beam. The optical probe includes a duct expansion part to expand the mammary duct when the optical probe is inserted into the mammary duct. An image signal processing unit is configured to output an image signal of the inside of the mammary duct using an interference signal generated using the response light beam and the reference light beam.

## Description

### BACKGROUND

### 1. Field

The following description relates to optical coherence tomography apparatuses, and more particularly, to optical coherence tomography apparatuses to diagnose breast cancer by inserting an optical probe into a mammary duct.

### 2. Description of the Related Art

Recently, methods and apparatuses to observe an internal structure of an organ, such as human tissue or any of various materials, have been widely used in various medical fields. Examples of the methods and apparatuses include various internal radiographic and tomography image capturing systems, such as X-ray systems, computerized tomography (CT) scanners, magnetic resonance imaging (MRI) systems, and ultrasound systems. These systems are important in a medical field because these systems enable medical practitioners to diagnose, perceive reasons, positions and progresses of various kinds of diseases without directly incising an internal organ of the human body or an organism. Thus, for such a diagnosis system, it is recognized that low harmfulness to an organism, high-resolution image acquisition, reasonable price, convenience in movement and usage, and the like are important features.

For instance, optical coherence tomography (OCT) apparatuses are devices capable of capturing an internal structure of an object through an interference phenomenon between light irradiated on and reflected by the object and reference light. Furthermore, because the OCT apparatuses can acquire high-resolution images and are harmless to the human body, the OCT apparatuses are widely used in the medical field. The OCT apparatuses were originally used for ophthalmologic diagnosis. The OCT apparatuses have expanded their application scope to cardiac and vascular diagnosis, tumor diagnosis, and so forth, and are also being widely used for tumor diagnosis of the gullet, the bronchus, and so forth at present.

### SUMMARY

Provided are optical coherence tomography apparatuses for diagnosing breast cancer.

Additional aspects will be set forth in part in the description which follows and, in part, will be apparent from the description, or may be learned by practice of the presented embodiments.

In accordance with an illustrative configuration there is provided an optical coherence tomography (OCT) apparatus for diagnosing breast cancer. The OCT apparatus includes an interference meter configured to split a light into a measurement light beam and a reference light beam to produce interference between the reference light beam and a response light beam. The response light beam includes the measurement light beam reflected inside of a mammary duct. An optical probe is configured to be inserted into the mammary duct, irradiate the measurement light beam inside the mammary duct, and receive the response light beam. The optical probe includes a duct expansion part to expand the mammary duct when the optical probe is inserted into the mammary duct. An image signal processing unit is configured to output an image signal of the inside of the mammary duct using an interference signal generated using the response light beam and the reference light beam.

The apparatus also includes a light-generating unit configured to generate the light; and a detection unit configured to detect the interference signal produced by the response light beam and the reference light beam.

The optical probe further includes a suction part configured to extract secretions inside the mammary duct when the optical probe is inserted into the mammary duct.

The apparatus also includes a position detection unit configured to detect a position of the optical probe when the optical probe is inserted into the mammary duct.

The position detection unit detects the position of the optical probe by irradiating ultrasound waves to the inside of a breast.

The optical probe further includes a radio frequency (RF) transmitter configured to transmit an RF signal, and the position detection unit includes an RF sensor disposed outside a breast and detects the position of the optical probe by receiving, through the RF sensor, the RF signal.

The position detection unit detects the position of the optical probe by irradiating X-rays inside of the breast and detecting X-rays transmitted through the breast.

The position detection unit includes a visible ray generation unit comprised in the optical probe and configured to inform a user of the position of the optical probe by generating a visible ray.

An end of the duct expansion part is formed in a spherical shape.

The optical probe includes: a sheath configured to protect an interior of the optical probe; and an optical fiber configured to output an optical signal inside the sheath. The interference meter and the optical probe are operatively connected to each other through the optical fiber. The optical fiber is formed of a flexible material to enable the optical fiber to be bent according to a shape of the mammary duct when the optical probe is inserted into the mammary duct, and is rotatable inside the sheath.

The optical probe includes a mirror configured to reflect the light changing a traveling direction of the light by 90°, and a rotation motor configured to rotate the mirror around an axis thereof to rotate a direction of the light irradiated outside of the optical probe.

In accordance with another configuration, there is provided a method of controlling an optical coherence tomography (OCT) apparatus for diagnosing breast cancer. The method includes inserting an optical probe into a mammary duct; expanding the mammary duct by expanding a duct expansion part of the optical probe; pushing the optical probe into the expanded mammary duct by contracting the duct expansion part; irradiating light inside the mammary duct from the optical probe; and generating a tomography image of the inside of the mammary duct using an interference signal generated from an interference caused between a reference light and a response light, wherein the response light includes the irradiated light reflected inside of the mammary duct.

The method also includes extracting secretions generated inside the mammary duct when the optical probe is inserted into the mammary duct.

The method also includes detecting a position of the optical probe when the optical probe is inserted into the mammary duct.

The detecting of the position of the optical probe includes detecting the position of the optical probe by irradiating ultrasound waves inside a breast.

The detecting of the position of the optical probe includes detecting the position of the optical probe by receiving a radio frequency (RF) signal transmitted by an RF transmitter in the optical probe.

The detecting of the position of the optical probe includes detecting the position of the optical probe by irradiating X-rays to the inside of the breast and detecting X-rays that have transmitted through the breast.

The detecting of the position of the optical probe includes detecting the position of the optical probe by detecting a visible ray generated by a visible ray generation unit in the optical probe.

The method also includes informing a user of the position of the optical probe by generating a visible ray through the optical probe.

In accordance with another configuration, there is provided a computer program embodied on a non-transitory computer-readable storage medium, the computer program being configured to control a processor to perform the method described above.

### BRIEF DESCRIPTION OF THE DRAWINGS

These and/or other aspects will become apparent and more readily appreciated from the following description of the embodiments, taken in conjunction with the accompanying drawings of which:

FIG. 1 is a block diagram of an optical coherence tomography (OCT) apparatus, according to an illustrative configuration;

FIG. 2 illustrates an example in which the OCT apparatus, according to an illustrative configuration, is applied to diagnose breast cancer;

FIGS. 3A to 3C illustrate sequential operations of inserting an optical probe of the OCT apparatus, according to an illustrative configuration, into a mammary duct;

FIGS. 4A and 4B are structures of the optical probe, according to illustrative configurations;

FIGS. 5A to 5D illustrate position detection units to detect a position of the optical probe, according to illustrative configurations;

FIG. 6 is a structure of the optical probe, according to another illustrative configuration; and

FIG. 7 is a flowchart illustrating a method to control the OCT apparatus, according to an illustrative configuration.

FIG. 8 is a flowchart illustrating another method to control an OCT apparatus, according to an illustrative configuration.

FIG. 9 is a flowchart illustrating a method to detect a position of an optical probe, according to an illustrative configuration.

### DETAILED DESCRIPTION

The following detailed description is provided to assist the reader in gaining a comprehensive understanding of the methods, apparatuses, and/or systems described herein. Accordingly, various changes, modifications, and equivalents of the systems, apparatuses and/or methods described herein will be suggested to those of ordinary skill in the art. Also, descriptions of well-known functions and constructions may be omitted for increased clarity and conciseness.

The terminology used herein is for the purpose of describing particular embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Example embodiments of the present invention are described herein with reference to cross-sectional illustrations that are schematic illustrations of idealized embodiments (and intermediate structures) of the present invention. As such, variations from the shapes of the illustrations as a result, for example, of manufacturing techniques and/or tolerances, are to be expected. Thus, example embodiments of the present invention should not be construed as limited to the particular shapes of regions illustrated herein but are to include deviations in shapes that result, for example, from manufacturing. The regions illustrated in the figures are schematic in nature and their shapes are not intended to illustrate the actual shape of a region of a device and are not intended to limit the scope of the present invention.

.

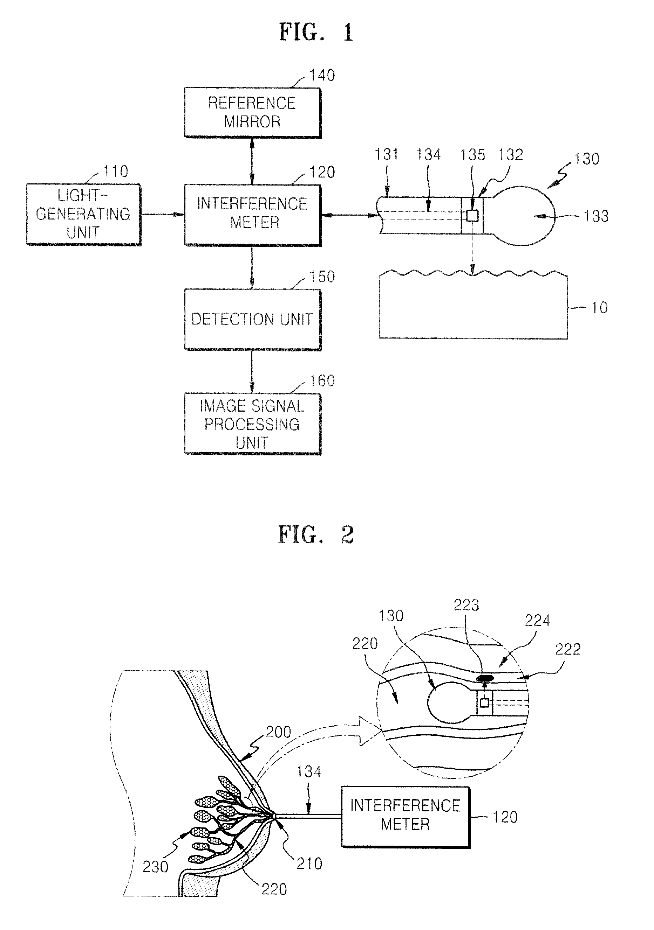

FIG. 1 is a block diagram of an optical coherence tomography (OCT) apparatus to diagnose breast cancer, according to an illustrative configuration. Referring to FIG. 1, the OCT apparatus may include an light-generating unit 110, an interference meter 120, an optical probe 130, a reference mirror 140, a detection unit 150, and an image signal processing unit 160. The OCT apparatus is suitable for inserting the optical probe 130 into the inside of the human body through a narrow entrance and capturing a tomography image of the inside of the human body. Thus, the OCT apparatus may capture a tomography image of internal tissue of a mammary duct of the breast by inserting the optical probe 130 into the mammary duct to diagnose breast cancer. Hereinafter, basic operational characteristics of the OCT apparatus according to an illustrative configuration will be described with reference to FIG. 1, and examples in which the OCT apparatus according to an illustrative configuration is applied to diagnose breast cancer will be described with reference to FIGS. 2 to 3C.

First, the basic operational characteristics of the OCT apparatus according to an illustrative configuration will now be described with reference to FIG. 1. The light-generating unit 110 generates light towards the interference meter 120. A beam splitter (not shown) included in the interference meter 120 splits the light delivered to the interference meter 120. Specifically, the beam splitter is an optical device, such as a reflection mirror, to reflect and transmit the light. The beam splitter in the interference meter 120 reflects a portion of the light delivered from the light-generating unit 110 and delivers the reflected portion of the light to the reference mirror 140. The reflected portion of the light delivered to the reference mirror 140 is then reflected by the reference mirror 140 and returned to the interference meter 120 as a reference light.

The beam splitter in the interference meter 120 reflects a remaining portion of the light delivered from the light-generating unit 110 and transmits the reflected remaining portion of the light to the optical probe 130 as a measurement light. The measurement light delivered to the optical probe 130 is irradiated through the optical probe 130 on an organ, a human body, a living organism, or an object 10 for which a tomography image of the inside thereof is to be captured. The object 10 reflects the irradiated measurement light as a response light. The response light is transmitted to the interference meter 120 via the optical probe 130.

The response light and the reference light reflected by the reference mirror 140 cause an interference in the interference meter 120, and the detection unit 150 detects an interference signal generated due to the interference occurring in the interference meter 120. The detection unit 150 transmits the detected interference signal to the image signal processing unit 160, and the image signal processing unit 160 outputs an image signal representing a tomography image of the object 10 using the received interference signal. The image signal processing unit 160 outputs the image signal to a display device (not shown), such as a monitor. The captured tomography image is displayed on the display device enabling a user to view the tomography image.

The optical probe 130 will now be described in more detail. In one illustrative example, the optical probe 130 includes a sheath 131, an optical fiber 134, a light-irradiating unit 135, a light-transmitting unit 132, and a duct expansion part 133. The OCT apparatus according to an illustrative configuration is designed to be suitable for the use in diagnosis of breast cancer or the like. The optical probe 130 has a feature of being easily inserted into a mammary duct of the breast.

The optical fiber 134 delivers the measurement light from the interference meter 120 to the light-irradiating unit 135. The optical fiber 134 is operatively connected to the interference meter 120 by extending from an interior of the optical probe 130 to the outside thereof. The optical fiber 134 may be formed of a flexible material to allow the optical fiber 134 to bend, when inserted into a mammary duct, according to a shape of the mammary duct, thereby reducing a pain of a patient.

The light-irradiating unit 135 irradiates the light delivered through the optical fiber 134 on the object 10. The light-irradiating unit 135 may include only a lens to directly irradiate the light delivered through the optical fiber 134 or may include the lens or lenses and a mirror to change a traveling direction of the light.

The interior of the optical probe 130 is isolated from the outside by the sheath 131 to prevent secretions inside the mammary duct from flowing into the interior of the optical probe 130 and to prevent debris from contaminating the inside of the mammary duct when a part, such as the optical fiber 134 or the light-irradiating unit 135, included in the optical probe 130 is broken.

The light-transmitting unit 132 transmits the light irradiated by the light-irradiating unit 135 to deliver the light to the outside of the optical probe 130. Thus, the light-transmitting unit 132 may be formed of a transparent material capable of transmitting light. The light-transmitting unit 132 may be formed in various forms, such as formed around the optical probe 130 so that the light-irradiating unit 135 can irradiate light by rotating 360°. In the alternative, the light-transmitting unit 132 may be formed on any one side of the optical probe 130.

The duct expansion part 133 allows the optical probe 130 to be easily inserted into a duct having a narrow diameter, such as the mammary duct. In detail, the duct expansion part 133 may expand or contract its volume. To insert the optical probe 130 into the mammary duct, the duct expansion part 133 is expanded to then expand the mammary duct. Once the mammary duct is expanded, the optical probe 130 may be easily inserted into the mammary duct. In process, the duct expansion part 133 contracts as the optical probe 130 continues to be inserted. When the optical probe 130 is completely inserted into the mammary duct, the optical probe 130 may be fixed inside the mammary duct by expanding the duct expansion part 133 to thereby easily capture a tomography image of the inside of the mammary duct.

In one example, the duct expansion part 133 may be implemented to be expanded or contracted using an air pressure. For example, the duct expansion part 133 may be formed of an elastic material so that a volume of the duct expansion part 133 expands when an internal air pressure increases and contracts when the internal air pressure decreases. However, the duct expansion part 133 is not limited thereto and may be implemented by other methods so that a user can control the volume of the duct expansion part 133 to expand or contract. In addition, the duct expansion part 133 may be formed in a spherical shape to thereby naturally expand the mammary duct when the optical probe 130 is initially inserted into the mammary duct and prevent an occurrence of perforation in the mammary duct.

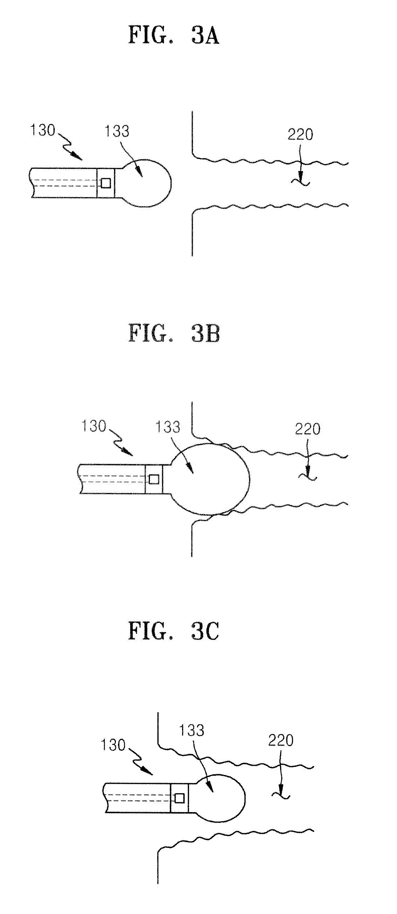

FIG. 2 illustrates an example in which the OCT apparatus according to an illustrative configuration is applied to diagnose breast cancer. Referring to FIG. 2, a mammary duct 220 and lobules 230 exist inside a breast 200. Each of the lobules 230 includes a number of mammary glands. The mammary duct 220 is a path through which mother's milk generated by the mammary glands of the lobules 230 flows, and the mammary duct 220 extends to a nipple 210 so that the mother's milk is discharged through the nipple 210. About 91% of breast cancer occurs in the mammary duct 220, but because a diameter of the mammary duct 220 is about 0.5 mm, it is not easy to photograph or examine the inside of the mammary duct 220. However, among other advantages, the optical probe 130 of the OCT apparatus according to an illustrative configuration, which is shown in FIG. 1, has an advantage that the optical probe 130 can be easily inserted into the mammary duct 220 using the duct expansion part 133. A detailed operation of inserting the optical probe 130 into the mammary duct 220 by controlling the duct expansion part 133 will be described below with reference to FIGS. 3A to 3C.

Referring back to FIG. 2, like other tissues, the mammary duct 220 includes an epithelium 222 and a dermis 224. FIG. 2 illustrates an example in which a tumor 223 exists in the epithelium 222. As such, when the tumor 223 exists only in the epithelium 222 of the mammary duct 220, the tumor 223 is called ductal carcinoma in situ (DCIS), and when the tumor 223 spreads to the dermis 224 through a basal membrane, the tumor 223 is called infiltrating (invasive) ductal carcinoma (IDC). Such DCIS, in which the tumor 223 exists only in the epithelium 222, is called stage zero (0) cancer, which is cancer that has hardly progressed. Meanwhile, when the tumor 223 is IDC, which has spread to the dermis 224, there is a risk that cancer cells may have spread along blood vessels in the dermis 224. Thus, it is very important to discover cancer in a DCIS stage, which is a stage before an IDC stage.

However, in a DCIS stage, a size of the tumor 223 is about 10 µm to about 30 µm, thereby making it difficult to diagnose the tumor 223 using ultrasound waves or X-rays with a resolution exceeding several hundred µm. In addition, when an endoscope is used, a user can only view the surface of the epithelium 222 of the mammary duct 220 and cannot view the inside of the epithelium 222. Thus, when the endoscope is used, only if a disorder occurs on the surface of the epithelium 222, the user can determine the presence of cancer through a separate biopsy. On the contrary, in accordance with some advantages, when breast cancer is diagnosed using the OCT apparatus according to an illustrative configuration, the breast cancer can be quickly and conveniently diagnosed at an early stage. In one example, because the OCT apparatus can obtain a tomography image up to a depth of about 2 mm, the whole epithelium 222 having a depth of about 1 mm to about 2 mm can be observed. Furthermore, because the OCT apparatus has a resolution of about 1 µm to about 10 µm, the OCT apparatus can discover the tumor 223 in an initial stage of cancer. In addition, a separate biopsy is not necessary because the tumor 223 inside the mammary duct 220 can be checked through the tomography image. Furthermore, safety is also secured because the diagnosis is performed using light that is harmless to the human body.

FIGS. 3A to 3C illustrate sequential operations of inserting the optical probe 130 of the OCT apparatus according to an illustrative configuration into the mammary duct 220. A process of inserting the optical probe 130 of the OCT apparatus according to an illustrative configuration into the mammary duct 220 will now be described in detail with reference to FIGS. 3A to 3C. FIG. 3A illustrates a figure immediately before the optical probe 130 is inserted into the mammary duct 220. When the optical probe 130 is initially inserted into the mammary duct 220, a volume of the duct expansion part 133 may be minimized so that the optical probe 130 can be easily inserted into a narrow entrance of the mammary duct 220. Because the duct expansion part 133 has a spherical shape and the volume is minimized, the optical probe 130 can be easily inserted into the mammary duct 220.

FIG. 3B illustrates a figure in which the mammary duct 220 is expanded by expanding the duct expansion part 133 immediately after the optical probe 130 is inserted into the mammary duct 220. The mammary duct 220 can be easily expanded by expanding the duct expansion part 133 after inserting the optical probe 130 into the mammary duct 220 in a state in which a volume of the duct expansion part 133 is minimized.

FIG. 3C illustrates a figure in which the optical probe 130 is inserted into the mammary duct 220 by contracting the duct expansion part 133 after the mammary duct 220 is expanded due to the expansion of the duct expansion part 133. The mammary duct 220 expanded in FIG. 3B maintains an expanded state than the normal state for a predetermined time for a moment in time after the duct expansion part 133 is contracted. As a result, the optical probe 130 can be easily inserted into the mammary duct 220 after contracting the duct expansion part 133.

As described with reference to FIGS. 3A to 3C, by repeating a process of expanding the duct expansion part 133 to expand the mammary duct 220 and contracting the duct expansion part 133 to insert the optical probe 130 into the mammary duct 220, the optical probe 130 can be easily inserted into the narrow inside of the mammary duct 220.

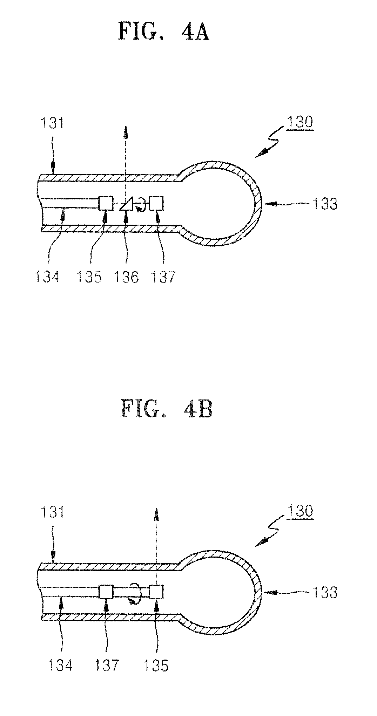

FIGS. 4A and 4B are structures of the optical probe 130, according to embodiments of the present invention. Referring to FIG. 4A, the optical probe 130 according to an illustrative configuration may include the optical fiber 134, the light-irradiating unit 135, a mirror 136, and a rotation motor 137 thereinside and include the sheath 131 and the duct expansion part 133. The mirror 136 reflects light irradiated by the light-irradiating unit 135 to change a traveling direction of the light by 90°. The rotation motor 137 may rotate the mirror 136 around an axis thereof by 360° to thereby rotate a direction of the light irradiated to the outside of the optical probe 130 by 360°.

Referring to FIG. 4B, the optical probe 130 according to another illustrative configuration may include the optical fiber 134, the light-irradiating unit 135, and the rotation motor 137 thereinside and include the sheath 131 and the duct expansion part 133. The rotation motor 137 rotates the light-irradiating unit 135 around the axis thereof by 360° to thereby rotate a direction of light irradiated to the outside of the optical probe 130 by 360°. Although only two embodiments are shown in FIGS. 4A and 4B, a structure of the optical probe 130 may be variously implemented.

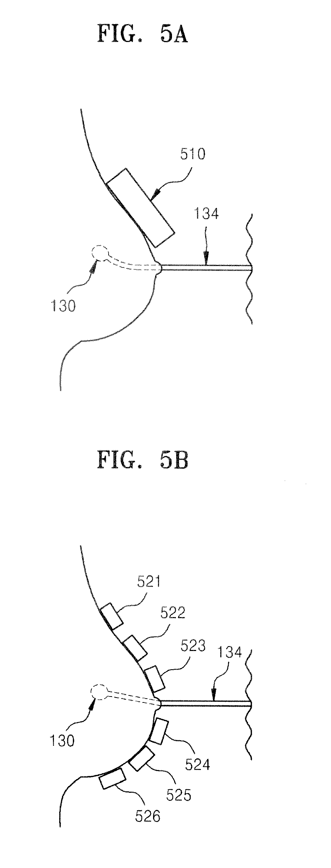

FIGS. 5A to 5D illustrate position detection units to detect a position of the optical probe 130, according to illustrative configurations. In detail, FIG. 5A illustrates a position detection unit to detect a position of the optical probe 130 using ultrasound waves, according to an illustrative configuration. The position of the optical probe 130 can be detected by irradiating ultrasound waves towards the breast using a ultrasound measurement device 510 external to the breast in a state where the optical probe 130 is inserted into a mammary duct,.

FIG. 5B illustrates a position detection unit for detecting a position of the optical probe 130 by using a radio frequency (RF) signal, according to an illustrative configuration. The optical probe 130 includes an RF transmitter (not shown), and RF receivers 521 to 526 are disposed outside the breast. When the RF transmitter included in the optical probe 130 transmits an RF signal, the RF receivers 521 to 526 disposed outside the breast receive the RF signal or RF signals, thereby detecting the position of the optical probe 130.

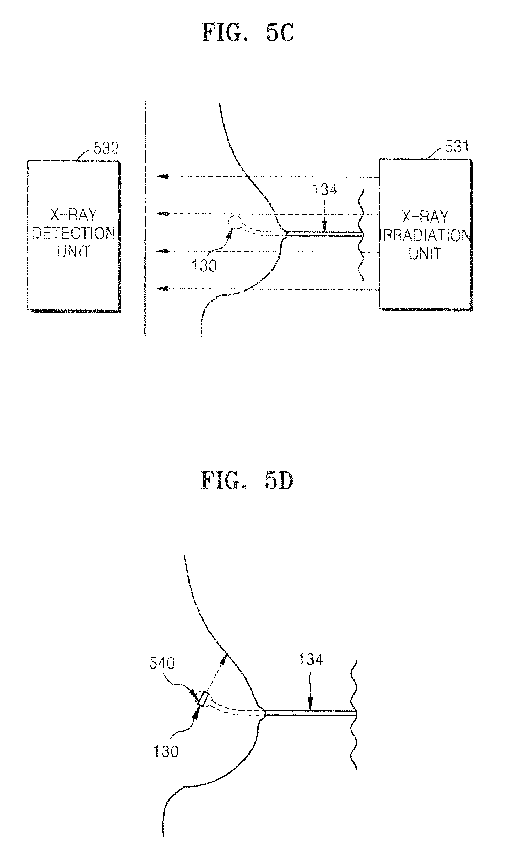

FIG. 5C illustrates a position detection unit to detect a position of the optical probe 130 by using X-rays, according to an illustrative configuration. An X-ray irradiation unit 531 is disposed at the front of the breast, and an X-ray detection unit 532 is disposed at the rear of the breast, that is, on the back of a patient. Thereafter, the position of the optical probe 130 can be detected by detecting X-rays irradiated by the X-ray irradiation unit 531 in the X-ray detection unit 532.

FIG. 5D illustrates a position detection unit to detect a position of the optical probe 130 by using a visible ray, according to an illustrative configuration. Referring to FIG. 5D, the optical probe 130 includes a visible ray generation unit 540. When the visible ray generation unit 540 generates a strong visible ray in a state where the optical probe 130 is inserted into a mammary duct, the position of the optical probe 130 can be detected by detecting the visible ray outside the breast. In this example, a visible ray having a specific color may be used to detect the visible ray outside the breast.

Because a position of the optical probe 130 cannot be perceived in a state where the optical probe 130 is inserted into the inside of a mammary duct, even though cancer is diagnosed, it is difficult to determine an accurate position of the cancer. However, by detecting a position of the optical probe 130 using various configurations as described above, an accurate position of the cancer can be detected.

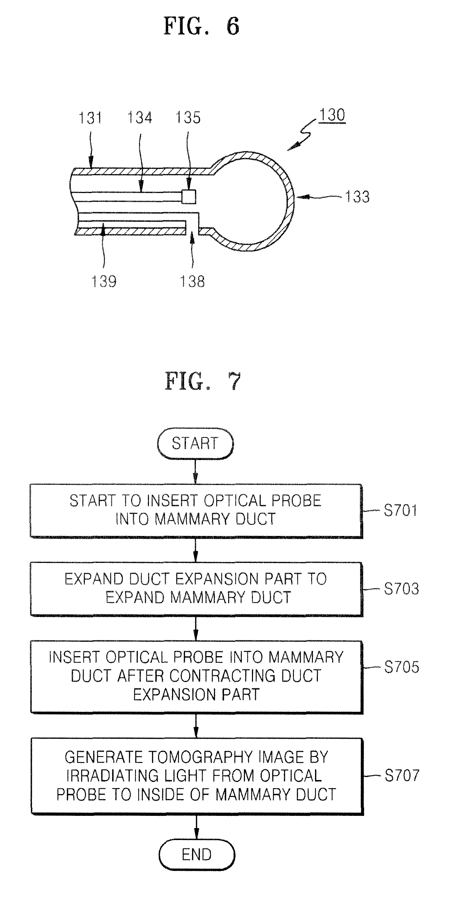

FIG. 6 is a structure of the optical probe 130, according to another illustrative configuration. Referring to FIG. 6, the optical probe 130 includes the optical fiber 134, the light-irradiating unit 135, the sheath 131, and the duct expansion part 133 similarly to the configurations described above and further includes a suction part 138. Secretions may exist inside a mammary duct and, because the secretions may obstruct tomography, the secretions may be extracted through the suction part 138. The secretions extracted through the suction part 138 may be delivered to the OCT apparatus through a suction tube 139, accumulated in the OCT apparatus, and discharged to the outside. As such, a relatively accurate tomography image can be acquired by extracting the secretions inside the mammary duct using the suction part 138.

FIG. 7 is a flowchart illustrating a method to control the OCT apparatus, according to an illustrative configuration. Referring to FIG. 7, at S701, an optical probe starts to be inserted into a mammary duct. When the optical probe is inserted into the mammary duct, at S703, a duct expansion part of the optical probe expands to expand the mammary duct. When the mammary duct is expanded, at S705, the optical probe is inserted into the mammary duct after contracting the duct expansion part. By inserting the optical probe into the expanded mammary duct, the optical probe can be easily inserted into the mammary duct. In addition, by starting to insert the optical probe into the mammary duct with a minimized volume of the duct expansion part, expanding the duct expansion part to expand the mammary duct, and contracting the duct expansion part again to more deeply insert the optical probe into the mammary duct, the optical probe can be easily inserted into the mammary duct. Also, a patient can suffer less pain due to the insertion. When the optical probe is completely inserted into the mammary duct, at S707, the optical probe irradiates light inside the mammary duct to generate a tomography image of the inside of the mammary duct.

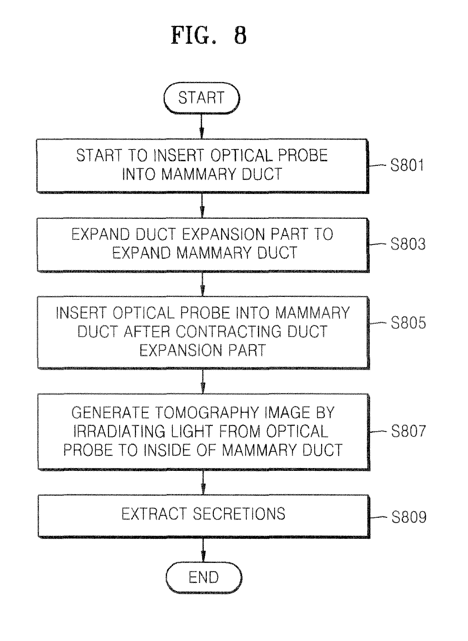

FIG. 8 is a flowchart illustrating another method to control an OCT apparatus, according to an illustrative configuration. Description of operations S801 to S807 correspond to operations S701 to S707, as described in reference to FIG. 7. In addition, at S809, secretions inside the mammary duct are extracted through the optical probe in the middle of the generation of the tomography image by inserting the optical probe into the inside of the mammary duct may be further included.

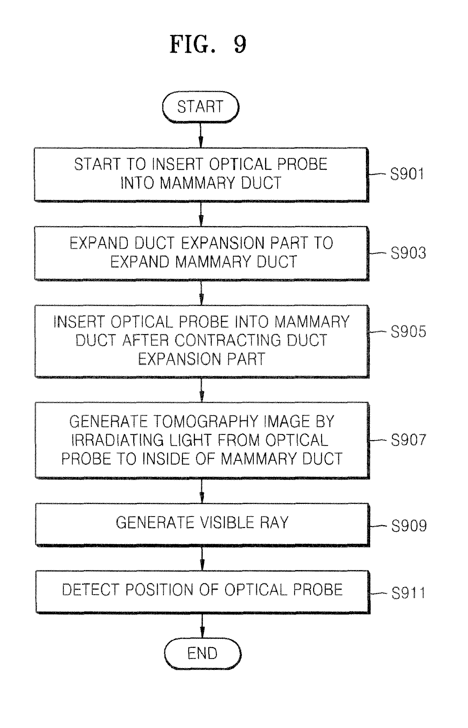

FIG. 9 is a flowchart illustrating a method to detect a position of an optical probe, according to an illustrative configuration. Description of operations S901 to S907 correspond to operations S701 to S707, as described in reference to FIG. 7. In addition, At S909, a strong visible ray is generated in a state where the optical probe is inserted into a mammary duct. At S911, the position of the optical probe is be detected by detecting the visible ray outside the breast using a radio frequency (RF) signal through at least one RF transmitter and at least one RF receiver outside the breast.

As described above, according to the one or more of the above embodiments of the present invention, by applying an OCT apparatus capable of acquiring high-resolution images of the inside of tissue to diagnosis of breast cancer, cancer can be diagnosed without a separate biopsy.

In addition, because an optical probe includes a duct expansion part capable of expanding and contracting its volume, the optical probe can be more easily inserted into a mammary duct, and a pain of a diagnosed patient can be reduced.

In addition, by detecting a position of the optical probe while the optical probe is being inserted into the mammary duct, an accurate occurrence point and/or location point of cancer can be perceived.

In addition, other embodiments of the present invention can also be implemented through computer-readable code/instructions in/on a medium, e.g., a computer-readable medium, to control at least one processing element to implement any above described embodiment. The medium can correspond to any medium/media permitting the storage and/or transmission of the computer-readable code.The units and apparatuses described herein may be implemented using hardware components. The hardware components may include, for example, controllers, sensors, processors, generators, drivers, and other equivalent electronic components. The hardware components may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The hardware components may run an operating system (OS) and one or more software applications that run on the OS. The hardware components also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a hardware component may include multiple processors or a processor and a controller. In addition, different processing configurations are possible, such a parallel processors.

The computer-readable code can be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more embodiments of the present invention. The media may also be a distributed network, so that the computer-readable code is stored/transferred and executed in a distributed fashion. Furthermore, the processing element could include a processor or a computer processor, and processing elements may be distributed and/or included in a single device.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. An optical coherence tomography, OCT, apparatus for diagnosing breast cancer, the OCT apparatus comprising:
an interference meter (120) configured to split a light into a measurement light beam and a reference light beam to produce interference between the reference light beam and a response light beam, wherein the response light beam comprises the measurement light beam reflected inside of a mammary duct (220);
an optical probe (130) configured to be inserted into the mammary duct, irradiate the measurement light beam inside the mammary duct, and receive the response light beam, wherein the optical probe comprises a duct expansion part to expand the mammary duct when the optical probe is inserted into the mammary duct; and
an image signal processing unit (160) configured to output an image signal of the inside of the mammary duct using an interference signal generated using the response light beam and the reference light beam.

2. The OCT apparatus of claim 1, further comprising:
a light-generating unit (110) configured to generate the light; and
a detection unit (150) configured to detect the interference signal produced by the response light beam and the reference light beam.

3. The OCT apparatus of claim 1 or 2, wherein the optical probe further comprises
a suction part (138) configured to extract secretions inside the mammary duct when the optical probe is inserted into the mammary duct.

4. The OCT apparatus of one of claims 1 to 3, further comprising:
a position detection unit configured to detect a position of the optical probe when the optical probe is inserted into the mammary duct.

5. The OCT apparatus of claim 4, wherein the position detection unit detects the position of the optical probe by irradiating ultrasound waves to the inside of a breast.

6. The OCT apparatus of claim 4, wherein the optical probe further comprises a radio frequency, RF, transmitter configured to transmit an RF signal, and
the position detection unit comprises an RF sensor disposed outside a breast and detects the position of the optical probe by receiving, through the RF sensor, the RF signal.

7. The OCT apparatus of claim 4, wherein the position detection unit detects the position of the optical probe by irradiating X-rays inside of the breast and detecting X-rays transmitted through the breast.

8. The OCT apparatus of claim 4, wherein the position detection unit comprises a visible ray generation unit comprised in the optical probe and configured to inform a user of the position of the optical probe by generating a visible ray.

9. The OCT apparatus of one of claims 1 to 8, wherein an end of the duct expansion part (133) is formed in a spherical shape.

10. The OCT apparatus of one of claims 1 to 9, wherein the optical probe (130) comprises:
a sheath (131) configured to protect an interior of the optical probe; and
an optical fiber (134) configured to output an optical signal inside the sheath,
wherein the interference meter (120) and the optical probe (130) are operatively connected to each other through the optical fiber (134), and
the optical fiber is formed of a flexible material to enable the optical fiber to be bent according to a shape of the mammary duct when the optical probe is inserted into the mammary duct, and is rotatable inside the sheath.

11. The OCT apparatus of one of claims 1 to 10, wherein the optical probe comprises:
a mirror (136) configured to reflect the light changing a traveling direction of the light by 90°, and
a rotation motor (137) configured to rotate the mirror around an axis thereof to rotate a direction of the light irradiated outside of the optical probe.

12. A method of controlling an optical coherence tomography, OCT, apparatus for diagnosing breast cancer, the method comprising:
inserting (S701) an optical probe into a mammary duct;
expanding (S703) the mammary duct by expanding a duct expansion part of the optical probe;
pushing (S705) the optical probe into the expanded mammary duct by contracting the duct expansion part;
irradiating light inside the mammary duct from the optical probe; and
generating (S707) a tomography image of the inside of the mammary duct using an interference signal generated from an interference caused between a reference light and a response light, wherein the response light comprises the irradiated light reflected inside of the mammary duct.

13. The method of claim 12, further comprising:
extracting secretions generated inside the mammary duct when the optical probe is inserted into the mammary duct.

14. The method of claim 12 or 13, further comprising:
detecting a position of the optical probe when the optical probe is inserted into the mammary duct.

15. A computer program embodied on a non-transitory computer-readable storage medium, the computer program being configured to control a processor to perform the method of any of claims 12 through 14.
